## Europäisches Patentamt

## European Patent Office

## Office européen des brevets

(11) Veröffentlichungsnummer: **0 014 920**
**B1**

(12) **EUROPÄISCHE PATENTSCHRIFT**

(45) Veröffentlichungstag der Patentschrift:
**08.12.82**

(21) Anmeldenummer: **80100651.1**

(22) Anmeldetag: **08.02.80**

(51) Int. Cl.³: **C 07 C 21/06, C 07 C 17/34**

(54) Verfahren zur Rückgewinnung von Pyrolyseenergie bei der Herstellung von Vinylchlorid durch unvollständige thermische Spaltung von 1,2-Dichloräthan.

(30) Priorität: 23.02.79 DE 2907066
31.03.79 DE 2913004
31.03.79 DE 2913030

(43) Veröffentlichungstag der Anmeldung:
03.09.80 Patentblatt 80/18

(45) Bekanntmachung des Hinweises auf die Patenterteilung:
08.12.82 Patentblatt 82/49

(84) Benannte Vertragsstaaten:
**BE DE FR GB IT NL**

(56) Entgegenhaltungen:
**DE-A-2 313 037**
**DE-B-1 250 426**

(73) Patentinhaber: **HOECHST AKTIENGESELLSCHAFT,
Postfach 80 03 20, D-6230 Frankfurt/Main 80 (DE)**

(72) Erfinder: **Czekay, Arno, Dr., Am Wachberg 50,
D-5042 Erftstadt (DE)**
Erfinder: **Höller, Ernst, Friedensweg 13,
D-5303 Bornheim-Merten (DE)**
Erfinder: **Kuxdorf, Bernhard, von Westerburg-Strasse 12,
D-5040 Brühl (DE)**
Erfinder: **Püsche, Herbert, Elsterweg 30,
D-5042 Erftstadt (DE)**
Erfinder: **Neuss, Hans-Wilhelm, Wittelsbacher
Strasse 14, D-5040 Brühl (DE)**
Erfinder: **Scholz, Harald, Dr., Am Beissel 8,
D-5042 Erftstadt (DE)**

## Verfahren zur Rückgewinnung von Pyrolyseenergie bei der Herstellung von Vinylchlorid durch unvollständige thermische Spaltung von 1,2-Dichloräthan

Die Herstellung von Vinylchlorid durch unvollständige thermische Spaltung von 1,2-Dichloräthan in einem Pyrolyseofen ist z. B. aus den DE-PS 857 957 und 899 191 sowie der US-PS 2 724 006 und der GB-PS 938 824 bekannt. Die gasförmigen Reaktionsprodukte der Pyrolyse, überwiegend Vinylchlorid, Chlorwasserstoff und nicht umgesetztes 1,2-Dichloräthan, welche den Pyrolyseofen bei Temperaturen von bevorzugt 480° bis 540°C unter einem Druck von 15 bis 25 bar und mit einem Wärmeinhalt von 0,3 bis 0,4 Giga-Joule (GJ) je 100 kg gewonnenes Vinylchlorid verlassen, wurden bisher hinter dem Pyrolyseofen in ein Quenchsystem mit direkter Kühlung (z. B. gemäß GB-PS 938 824) oder durch einen Kühler mit indirekter Kühlung (z. B. gemäß US-PS 2 724 006 oder 3 476 955) geleitet, wobei durch Wärmeabfuhr Total- oder Teilkondensation eintritt.

Die Spaltgase stellen infolge ihres Gehalts an Chlorwasserstoff und Kohlenstoffteilchen sowie wegen ihrer hohen Temperaturen und Drücke ein aggressives Medium dar, dessen Wärmeinhalt bisher ungenutzt an das Kühlmedium Luft oder Wasser abgegeben wurde.

Die kondensierten Spaltgase werden einer Destillationsstraße zugeführt, worin die Auftrennung in die einzelnen Bestandteile erfolgt. Die Destillationsstraße besteht aus Kolonnen, in denen nacheinander der Chlorwasserstoff, das Vinylchlorid und die unter und über 83,7°C (1011 mbar) siedenden Verunreinigungen vom nicht umgesetzten 1,2-Dichloräthan abgetrennt werden (Leichtsieder- und Hochsiederkolonne). Wie US-PS 3 476 955 zeigt, kann auf eine Leichtsiederkolonne auch verzichtet werden.

Die Erfindung betrifft nun ein Verfahren zur Rückgewinnung von Pyrolyseenergie bei der Herstellung von Vinylchlorid, wobei man flüssiges 1,2-Dichloräthan unter Druck vorwärmt, verdampft und gasförmig einer Spaltzone zuführt, nach erfolgter unvollständiger thermischer Spaltung die unter Druck stehenden heißen Spaltgase kühlt und nacheinander in mehreren Destillationszonen zur Entfernung von Chlorwasserstoff, Vinylchlorid, ggf. Leichtsiedern und von Hochsiedern unter Rückgewinnung des nichtumgesetzten 1,2-Dichloräthans in ihre Bestandteile auftrennt, welches dadurch gekennzeichnet ist, daß man die heißen Spaltgase in einem Wärmetauscher kühlt, der mantelseitig

a) vom Sumpf einer Destillationszone als Kühlmittel im Kreislauf durchströmt wird und dabei im Wärmetausch die betreffende Destillationszone heizt, oder

b) von einem im Kreislauf von einem Umlaufverdampfer mindestens einer Destillationszone herangeführten Wärmeübertragungsmittel durchströmt wird und dabei im Wärmeaustausch den Umlaufverdampfer mindestens einer Destillationszone beheizt, oder

c) von flüssigem 1,2-Dichloräthan als Kühlmittel durchströmt wird, das nach Wärmeaufnahme gasförmig der Spaltzone zugeführt wird.

Überraschenderweise wurde festgestellt, daß gemäß der Erfindung die für die Spaltung von 1,2-Dichloräthan aufgewendete Wärmemenge — soweit sie als Wärmeinhalt der Spaltgase auftritt — nutzbringend zurückgewonnen werden kann, indem man damit die Destillationskolonnen für die Auftrennung der eigenen Spaltprodukte heizt. Zu diesem Zweck wird das Spaltgas zuerst einem oder mehreren Wärmetauschern zugeführt, die mantelseitig mit dem Blaseninhalt je einer Destillationskolonne als Kühlmittel beaufschlagt sind. Der Blaseninhalt besteht dabei überwiegend aus rohem 1,2-Dichloräthan. Praktisch also ein mit Spaltgas beschickter Wärmetauscher die Funktion eines Umlaufverdampfers für die Chlorwasserstoff-, Vinylchlorid-, Leichtsieder- oder Hochsiederkolonne.

Überraschenderweise wurde weiterhin festgestellt, daß die gefährlichen Eigenschaften des wärmeabgebenden Spaltgases, besonders die hohe Temperatur und die mitgeführten Kohlenstoffteilchen, das Betreiben eines Wärmetauschers nicht ausschließen. Die hohe Eintrittstemperatur der Spaltgase, die eine geringfügige Spaltung des mantelseitigen Roh-Dichloräthans hervorruft, erzeugt keine Produkte, die im Rohdichloräthan nicht ohnehin vorhanden sind. Derselbe Vorteil gilt im Falle von Undichtigkeiten im Wärmetauscher; da es sich produkt- wie mantelseitig praktisch um dieselben Stoffe handelt, sind explosionsartige Reaktionen, wie z. B. die Bildung von Salzsäure bei der Kühlung mit Wasser, nicht zu erwarten. Der im Spaltgasstrom mitgeführte Kohlenstoff scheidet sich nur in geringem Maße im Wärmetauscher ab und seine Isolationswirkung kann wegen der hohen Temperaturdifferenz zwischen Produkt- und Mantelseite vernachlässigt werden. Allerdings muß die Reaktion im Spaltofen so geführt werden, daß wenig Ruß erzeugt wird. Dafür ist es z. B. vorteilhaft, das zur Spaltung vorgesehene 1,2-Dichloräthan dem Ofen gasförmig zuzuführen.

Die als Umlaufverdampfer wirkenden Wärmetauscher sind zweckmäßig so gebaut, daß das Spaltgas als wärmeabgebendes Medium durch die Rohre geführt und das wärmeaufnehmende Medium, d. h. der Blaseninhalt einer Destillationskolonne, innerhalb des Mantels um die Rohre geleitet wird. Man kann Rohrbündelwärmeaustauscher oder besser noch Einrohrapparate einsetzen, deren Rohrführung mäanderförmig in einem Gehäuse angeordnet ist.

Es ist auch möglich, die heißen Spaltgase in mehreren hintereinandergeschalteten Wärmetauschern zu kühlen, die mantelseitig vom Sumpf je einer Destillationszone als Kühlmittel im Kreislauf durchströmt werden und dabei im Wärmeaustausch die betreffende Destillationszone heizen.

Bevorzugt durchströmt das Sumpfprodukt einer Destillationszone den Wärmetauscher mantelseitig unter einem Druck von 1–15 bar mit etwa Siedetemperatur, verdampft infolge Wärmeaufnahme seitens der Spaltgase und strömt dampfartig in die Destillationszone zurück.

Die heißen Spaltgase durchströmen den oder die Wärmetauscher produktseitig vorzugsweise mit einer Geschwindigkeit von mindestens 20 m/s.

Im Falle der Verwendung von nur einem Wärmetauscher, der mantelseitig mit dem Blaseninhalt von nur einer Destillationskolonne als Kühlmedium beschickt wird, ist das Spaltgas nach Verlassen dieses Wärmetauschers im allgemeinen noch zu heiß, um in die Destillationskolonne zur Abtrennung von Chlorwasserstoff eingeleitet zu werden. Man führt die Spaltgase daher noch durch ein Quenchsystem, worin sie mit im Kreislauf geführtem, verflüssigten Spaltgas gewaschen und gekühlt werden, wobei die Verunreinigungen an festem Kohlenstoff in die Flüssigphase gelangen und abfiltriert werden können. Diese Quenche kann wesentlich kleiner als üblich sein, da ihr ein Wärmetauscher vorgeschaltet ist.

Im Falle der Verwendung von zwei hintereinandergeschalteten Wärmetauschern, die mantelseitig mit je einem Blaseninhalt von insgesamt zwei Destillationskolonnen als Kühlmedien beschickt werden, wird die Pyrolyseenergie so weit wie möglich zurückgewonnen, so daß die Nachschaltung selbst einer kleinen Quenche unnötig ist. Doch ist es zweckmäßig, statt dessen einen Kondensator nachzuschalten, um die abgekühlten Spaltgase zwecks Auswaschung von festem Kohlenstoff teilweise zu verflüssigen.

Gemäß einer zweiten Variante der Erfindung wird das Spaltgas zuerst einem Wärmetauscher zugeführt, der mantelseitig mit einem Wärmeübertragungsmittel beaufschlagt wird. Dieses Wärmeübertragungsmittel zirkuliert im geschlossenen Kreislauf zwischen dem oder den Umlaufverdampfern einer oder mehrerer Destillationskolonnen und dem Mantelraum des Wärmetauschers. Somit können mehrere Umlaufverdampfer mit Hilfe des einen Wärmetauschers beheizt werden. Praktisch übernimmt also ein produktseitig mit Spaltgas beschickter Wärmetauscher die Beheizung eines oder mehrerer Umlaufverdampfer für die Chlorwasserstoff-, Vinylchlorid-, Leichtsieder- oder Hochsiederkolonne.

Der als Heizung für die Umlaufverdampfer wirkende Wärmetauscher ist zweckmäßig so gebaut, daß das Spaltgas als wärmeabgebendes Fluid durch die Rohre geführt und das wärmeaufnehmende Fluid, d. h. das Wärmeübertragungsmittel, innerhalb des Mantels um die Rohre geleitet wird. Man kann Rohrbündelwärmeaustauscher oder besser noch Einrohrapparate einsetzen, deren Rohrführung mäanderförmig in einem Gehäuse angeordnet ist.

Als Wärmeübertragungsmittel setzt man vorzugsweise Wasser ein.

Bevorzugt durchströmt das vom Umlaufverdampfer einer Destillationszone als Wärmeübertragungsmittel herangeführte Wasser den Wärmetauscher mantelseitig unter einem Druck von 5 bis 20 bar mit etwa Siedetemperatur, verdampft infolge Wärmeaufnahme seitens der Spaltgase und strömt dampfförmig zum Umlaufverdampfer der Destillationszone zurück.

Statt Wasser kann man auch ein Wärmeübertragungsmittel mit im Vergleich zu Wasser niedrigerem Dampfdruck und höherer kritischer Temperatur ($> 374,1° C$) einsetzen.

Als Wärmeübertragungsmittel können die in Römpps Chemie-Lexikon 6. Auflage, Spalten 7055—7056, 7. Auflage, Seite 3863 sowie in W. Wagner, Wärmeträgertechnik mit organischen Medien, 3. Auflage (1977), Technischer Verlag Resch KG, 8032 Gräfelfing, genannten Stoffe verwendet werden, so z. B. flüssiges Natrium, Natrium/Kalium-Legierungen, Quecksilber, Rosesches und Woodsches Metall, Salzschmelzen, z. B. eutektische Gemische aus $KNO_3$, $NaNO_3$ und $NaNO_2$, Wärmeübertragungsöle auf der Basis von Siliconen, Biphenylen, Diphenyläthern, Diphenylmethanderivaten, aliphatischen Mineralölen, Polyalkylenglykolen, Diphenyloxid, Ditolyläthern. Bewährt hat sich besonders Marlotherm[(R)] S; (R) = eingetragenes Warenzeichen der Chemische Werke Hüls AG, Marl. Es hat einen Siedepunkt von 390°C und besteht nach Römpps Chemie-Lexikon, 7. Auflage (1974), Seite 2075 sowie nach W. Wagner, a.a.O. Seite 61, aus einem Gemisch isomerer Dibenzylbenzole.

Die heißen Spaltgase durchströmen den Wärmetauscher produktseitig, vorzugsweise mit einer Geschwindigkeit von mindestens 20 m/s.

Die DE-AS 23 13 037 beschreibt ebenfalls ein Verfahren zur Herstellung von Vinylchlorid durch unvollständige thermische Spaltung von 1,2-Dichloräthan. Hierbei wird flüssiges 1,2-Dichloräthan zunächst unter einem Druck von 21 bis 31 bar auf 180 bis 200°C erwärmt, das erwärmte flüssige Produkt wird filtriert, teilweise verdampft und gasförmig in den Spaltreaktor geleitet. Die Vorwärmzone befindet sich dabei über der Spaltzone in derselben Ofeneinheit und wird von derselben Wärmequelle beheizt. Nach einer anderen Variante kann man das flüssige 1,2-Dichloräthan auch ohne gesonderte Vorwärmung unter einem Druck von 21 bis 36 bar und bei einer Temperatur von 200 bis 250°C teilweise verdampfen und den gasförmigen Anteil dem Spaltreaktor zuführen, wobei die Verdampfungszone über der Spaltzone in derselben Ofeneinheit angeordnet sein und von derselben Wärmequelle beheizt werden kann.

Überraschenderweise wurde festgestellt, daß gemäß einer dritten Variante der Erfindung die für die Spaltung von 1,2-Dichloräthan aufgewendete Wärmemenge — soweit sie als Wärmeinhalt der Spaltgase auftritt — nutzbringend zurückgewonnen werden kann, indem man damit das flüssige 1,2-Dichloräthan vorwärmt oder verdampft, bevor es gasförmig in die Spaltzone geleitet wird. Zu diesem Zweck wird das Spaltgas zuerst einem Wärmetauscher zugeführt, der mantelseitig mit, ggf. vorgewärmtem, flüssigen 1,2-Dichloräthan als Kühlmittel beaufschlagt ist. Praktisch übernimmt also ein mit Spaltgas beschickter Wärmetauscher die Funktion eines Verdampfers oder eines Vorwärmers für das thermisch zu spaltende 1,2-Dichloräthan.

Der als Verdampfer oder Vorwärmer wirkende Wärmetauscher ist zweckmäßig so gebaut, daß das Spaltgas als wärmeabgebendes Fluid durch die Rohre geführt und das wärmeaufnehmende Fluid, d. h. das flüssige, ggf. vorgewärmte 1,2-Dichloräthan, innerhalb des Mantels um die Rohre geleitet wird. Man kann Rohrbündelwärmeaustauscher oder besser noch Einrohrapparate einsetzen, deren Rohrführung mäanderförmig in einem Gehäuse angeordnet ist.

Das Verfahren der dritten Variante der Erfindung kann ferner wahlweise und bevorzugt dadurch gekennzeichnet sein, daß

a) man das zu spaltende flüssige 1,2-Dichloräthan unter einem Druck von 27 bis 36 bar auf 225 bis 255°C vorwärmt, noch flüssig als Kühlmittel in die Mantelzone des Wärmetauschers schickt und gasförmig unter einem Druck von 26 bis 35 bar mit einer Temperatur von 240 bis 260°C abzieht und der Spaltzone zuführt;

b) man das zu spaltende flüssige 1,2-Dichloräthan unter einem Druck von 30 bis 40 bar und mit einer Temperatur von 30 bis 50°C als Kühlmittel in die Mantelzone des Wärmetauschers schickt, dort vorwärmt, in einer anschließenden Verdampferzone verdampft und gasförmig unter einem Druck von 27 bis 36 bar mit einer Temperatur von 225 bis 255°C der Spaltzone zuführt;

c) die heißen Spaltgase den Wärmetauscher produktseitig mit einer Geschwindigkeit von mindestens 20 m/s durchströmen.

Das Spaltgas ist nach Verlassen des Wärmetauschers im allgemeinen noch zu heiß, um in die Destillationskolonne zur Abtrennung von Chlorwasserstoff eingeleitet zu werden. Man führt die Spaltgase daher noch durch ein Quenchsystem, worin sie mit im Kreislauf geführtem, verflüssigten Spaltgas gewaschen und gekühlt werden, wobei die Verunreinigungen an festem Kohlenstoff in die Flüssigphase gelangen und abfiltriert werden können. Diese Quenche kann wesentlich kleiner als üblich sein, da ihr ein Wärmetauscher vorgeschaltet ist.

Im übrigen kann die Aufarbeitung des den Wärmetauscher oder die nachgeschaltete Quenche verlassenden Spaltgases unter Gewinnung von Chlorwasserstoff und Vinylchlorid sowie Rückgewinnung von nicht-umgesetztem 1,2-Dichloräthan in bekannter Weise, z. B. entsprechend den in den DE-PS 1 242 594 und 1 250 426 beschriebenen Verfahrensweisen erfolgen.

### Beispiel 1

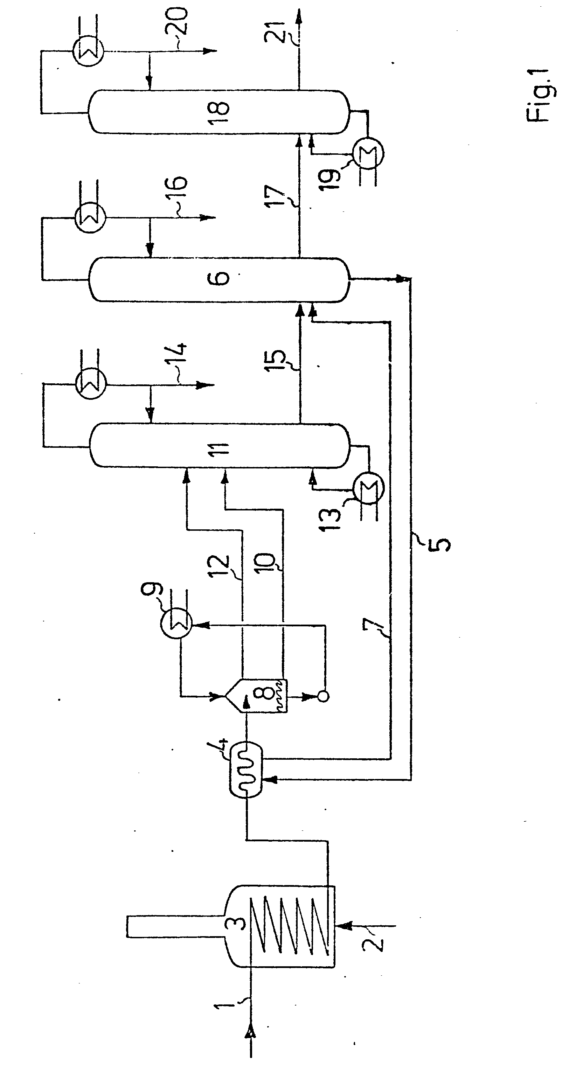

Man arbeitet gemäß dem Fließschema der Figur 1.

Über Leitung (1) wird dem mit Brenngas aus Leitung (2) beheizten Spaltofen (3) 1,2-Dichloräthan gasförmig zugeführt. 38 000 kg/h Spaltgas verlassen den Spaltofen und treten mit einer Geschwindigkeit von 30 m/s, einer Temperatur von 500°C und einem Druck von 23 bar in den Wärmetauscher (4) ein. Dieser Wärmetauscher (4) wird mantelseitig mit über Leitung (5) herangeführtem, aus rohem 1,2-Dichloräthan bestehenden Sumpfprodukt (Temperatur: 148°C) der Vinylchloridkolonne (6) als wärmeabführendes Kühlmedium, welches unter einem Druck von 5—6 bar steht und bei 145° bis 155°C verdampft, beschickt. Hierdurch werden die Spaltgase auf 280°C (20 bar) abgekühlt, während das Kühlmedium dampfförmig über Leitung (7) in die Blase der Kolonne (6) zurückgeführt wird. Auf diese Weise wird im Wärmetauscher (4) eine Wärmemenge von 10,5 GJ/h ausgetauscht, was einer Heizdampfmenge von etwa 5 t/h entspricht, die bisher zum Betreiben der Kolonne (6) erforderlich war.

Die 280°C heißen Spaltgase gelangen sodann in die Quenche (8), wo sie direkt mit gekühltem Kondensat (Temperatur: 55°C) abgeschreckt und teilkondensiert werden. Ein konstanter Anteil des Kondensats wird über den Kühler (9) im Kreislauf auf den Kopf der Quenche (8) zurückgeführt, während der größere Teil über Leitung (10) in die Chlorwasserstoffkolonne (11) gelangt. Die in der Quenche (8) nicht kondensierten Anteile des Spaltgases werden der Kolonne (11) über Leitung (12) zugeführt. Die Kolonne (11) wird mit dem Umlaufverdampfer (13) beheizt. Chlorwasserstoff wird über Leitung (14) abgetrennt. Das restliche Kondensat gelangt über Leitung (15) in die Vinylchloridkolonne (6), deren Sumpf mit dem Wärmeaustauscher (4) in direkter fließender Verbindung steht und von diesem beheizt wird. Vinylchlorid wird über Leitung (16) abgetrennt. Das zurückbleibende, nicht umgesetzte Rohdichloräthan wird über die Leitung (17) ohne Zwischenschaltung einer Leichtsiederkolonne direkt der Hochsiederkolonne (18) zugeführt, welche mit Umlauferhitzer (19) beheizt wird. Über Leitung (20) werden 1,2-Dichloräthan und über Leitung (21) die Hochsieder abgezogen.

### Beispiel 2

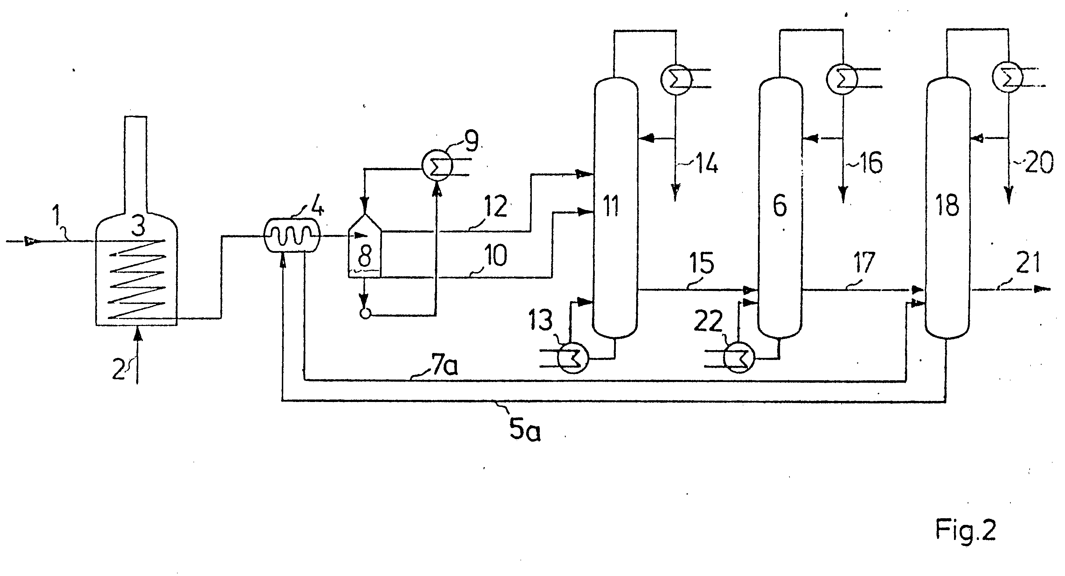

Man arbeitet gemäß dem Fließschema der Figur 2 und verfährt somit im wesentlichen wie im Beispiel 1. Das Kühlmedium für die mantelseitige Beschickung des Wärmetauschers (4) wird jedoch über Leitung (5a) dem Sumpf der Hochsiederkolonne (18) entnommen. Dieses Kühlmedium hat eine Temperatur von 95°C und steht unter einem Druck von 1,2—1,8 bar. Es siedet bei 92—105°C und wird dampfförmig über die Leitung (7a) im Kreislauf in die Blase der Kolonne (18) zurückgeführt. Der Spaltgasstrom wird im Wärmetauscher (4) von 500° auf 195°C abgekühlt, wobei eine Wärmemenge von 14 GJ/h entsprechend einer Heizdampfmenge von 6,7 t/h ausgetauscht wird.

Die Hochsieder-Kolonne wird somit nicht mehr durch einen Umlaufverdampfer (Fig. 1, [19]) mittels Heizdampf, sondern über die Leitungen (5a) und (7a) mit Hilfe der Pyrolyseenergie der Spaltgase betrieben. Gegenüber Fig. 1 wird jedoch die Vinylchloridkolonne (6) jetzt durch den Umlaufverdampfer (22) beheizt.

### Beispiel 3

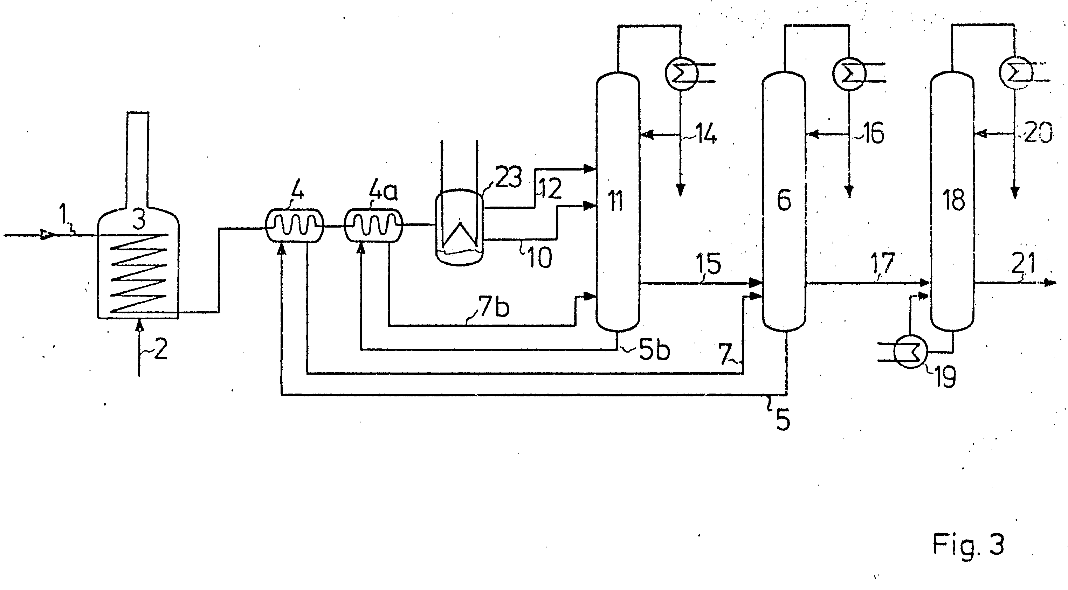

Man arbeitet gemäß dem Fließschema der Figur 3 und verfährt somit im wesentlichen wie im Beispiel 1. Hinter den Wärmetauscher (4) wird jedoch noch ein weiterer Wärmetauscher (4a) geschaltet, der mantelseitig über Leitung (5b) mit dem Sumpfprodukt (Temperatur 94°C) der HCl-Kolonne (11) beschickt wird, welches unter einem Druck von 12,5 bar steht und bei 94°C siedet. Es besteht überwiegend aus 1,2-Dichloräthan und Vinylchlorid und wird dampfförmig im Kreislauf über Leitung (7b) in die Blase der Kolonne (11) zurückgeführt. Im Wärmetauscher (4a) werden die Spaltgase von 280° auf 180°C abgekühlt. Dabei wird zusätzlich eine Wärmemenge von 4,2 GJ/h entsprechend einer Heizdampfmenge von etw 2 t/h übertragen. Der gesamte Wärmeaustausch beträgt somit 14,7 GJ/h entsprechend einer Heizdampfmenge von etwa 7 t/h.

Die HCl-Kolonne (11) wird demnach nicht mehr durch einen Umlaufverdampfer (Fig. 1, [13]) mittels Heizdampf, sondern über die Leitungen (5b) und (7b) mit Hilfe der Pyrolyseenergie der Spaltgase betrieben. Anstelle der kleinen Quenche (8) mit Kühler (9) aus Figur 1 (Beispiel 1) wird nunmehr im Zuge einer weiteren Einsparungsmöglichkeit ein kleiner Kondensator (23) gesetzt, der eine teilweise Kondensation der Spaltgase ermöglicht.

### Beispiel 4

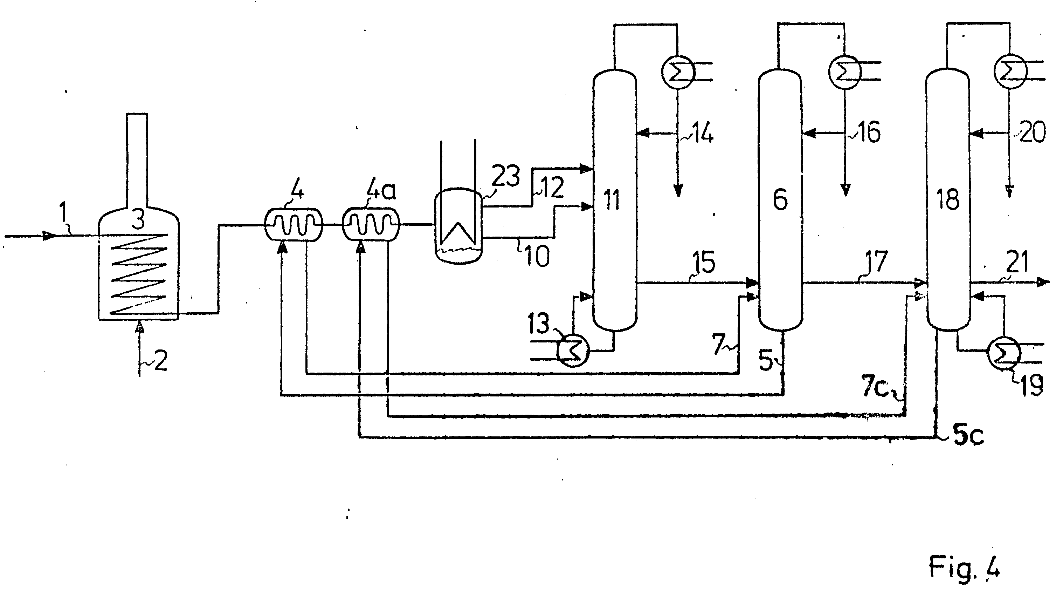

Man arbeitet gemäß dem Fließschema der Figur 4 und verfährt somit im wesentlichen wie im Beispiel 1. Hinter den Wärmetauscher (4) wird jedoch noch ein weiterer Wärmetauscher (4a) geschaltet, der mantelseitig über Leitung (5c) mit dem Sumpfprodukt (Temperatur 95°C) der Hochsiederkolonne (18) beschickt wird, welches unter einem Druck von 1,2—1,8 bar steht und bei 92—105°C siedet. Es wird über Leitung (7c) dampfförmig in die Blase der Kolonne (18) zurückgeführt. Im Wärmetauscher (4a) werden die Spaltgase von 280° auf 120°C abgekühlt und dabei teilkondensiert. In der Hochsiederkolonne (18) können auf diese Weise etwa 80% des normalerweise erforderlichen Heizdampfes ersetzt werden, so daß die insgesamt eingesparte Heizdampfmenge 9 t/h entsprechend 18,9 GJ/h zurückgewonnener Pyrolyseenergie beträgt. Der restliche Wärmebedarf der Hochsiederkolonne (18) wurde mit dem Umlaufverdampfer (19) durch Heizdampf gedeckt.

Anstelle der kleinen Quenche (8) mit Kühler (9) aus Figur 1 (Beispiel 1) wird nunmehr im Zuge einer weiteren Einsparungsmöglichkeit ein kleiner Kondensator (23) gesetzt, der eine weitere Kondensation der bereits teilweise kondensierten Spaltgase ermöglicht.

### Beispiel 5

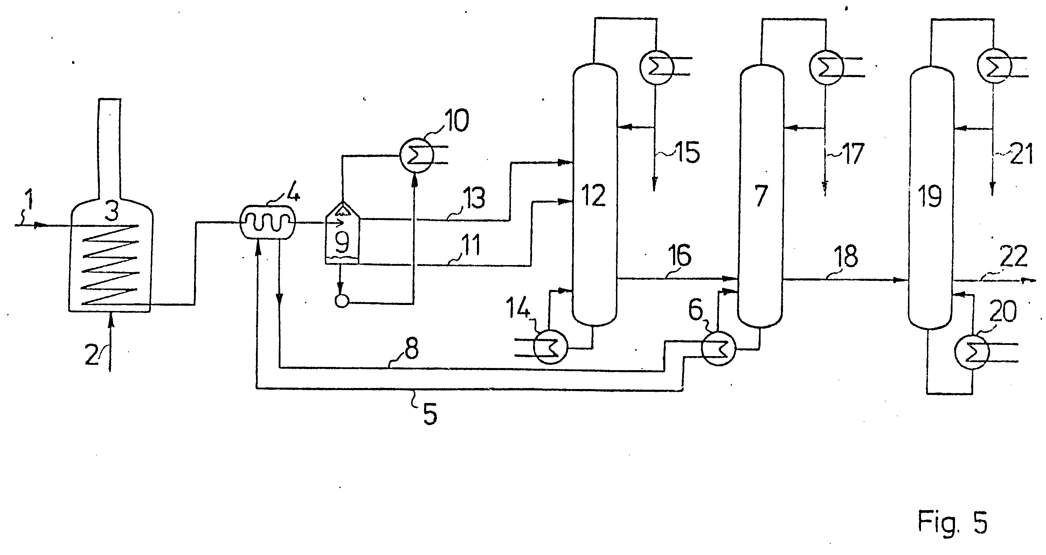

Man arbeitet gemäß dem Fließschema der Figur 5.

Über Leitung (1) wird dem mit Brenngas aus Leitung (2) beheizten Spaltofen (3) 1,2-Dichloräthan gasförmig zugeführt. 38 000 kg/h Spaltgas verlassen den Spaltofen und treten mit einer Geschwindigkeit von 30,6 m/s, einer Temperatur von 510°C und einem Druck von 21 bar in den Wärmetauscher (4) ein. Dieser Wärmetauscher (4) wird mantelseitig mit über Leitung (5) vom Umlaufverdampfer (6) der Vinylchloridkolonne (7) als wärmeabführendem Kühlmedium herangeführtem Wasser (Temperatur 201°C), welches unter einem Druck von 16 bis 17 bar steht und bei 201° bis 204°C verdampft, beschickt. Hierdurch werden die Spaltgase auf 290°C (18 bar) abgekühlt, während das Kühlmedium in Form von Wasserdampf über Leitung (8) zum Umlaufverdampfer (6) zurückgeführt wird. Auf diese Weise wird im Wärmetauscher (4) eine Wärmemenge von 10,5 GJ/h ausgetauscht, was einer Heizdampfmenge von etwa 5 t/h entspricht, die bisher zum Betreiben der Kolonne (7) erforderlich war.

Die 290°C heißen Spaltgase gelangen sodann in die Quenche (9), wo sie direkt mit gekühltem Kondensat (Temperatur: 55°C) abgeschreckt und teilkondensiert werden. Ein konstanter Anteil des Kondensats wird über den Kühler (10) im Kreislauf auf den Kopf der Quenche (9) zurückgeführt, während der größere Teil über Leitung (11) in die Chlorwasserstoffkolonne (12) gelangt. Die in der Quenche (9) nicht kondensierten Anteile des Spaltgases werden der Kolonne (12) über Leitung (13) zugeführt. Die Kolonne (12) wird mit dem Umlaufverdampfer (14) beheizt. Chlorwasserstoff wird über Leitung (15) abgetrennt. Das restliche Kondensat gelangt über Leitung (16) in die Vinylchloridkolonne (7), deren Sumpf mit dem Wärmeaustauscher (4) indirekt in Verbindung steht und von diesem via Umlaufverdampfer (6) beheizt wird. Vinylchlorid wird über Leitung (17) abgetrennt. Das zurückbleibende, nicht umgesetzte Rohdichloräthan wird über die Leitung (18) ohne Zwischenschaltung einer Leichtsiederkolonne direkt der Hochsiederkolonne (19) zugeführt, welche mit Umlauferhitzer (20) beheizt wird. Über Leitung (21) werden 1,2-Dichloräthan, über Leitung (22) die Hochsieder abgezogen.

### Beispiel 6

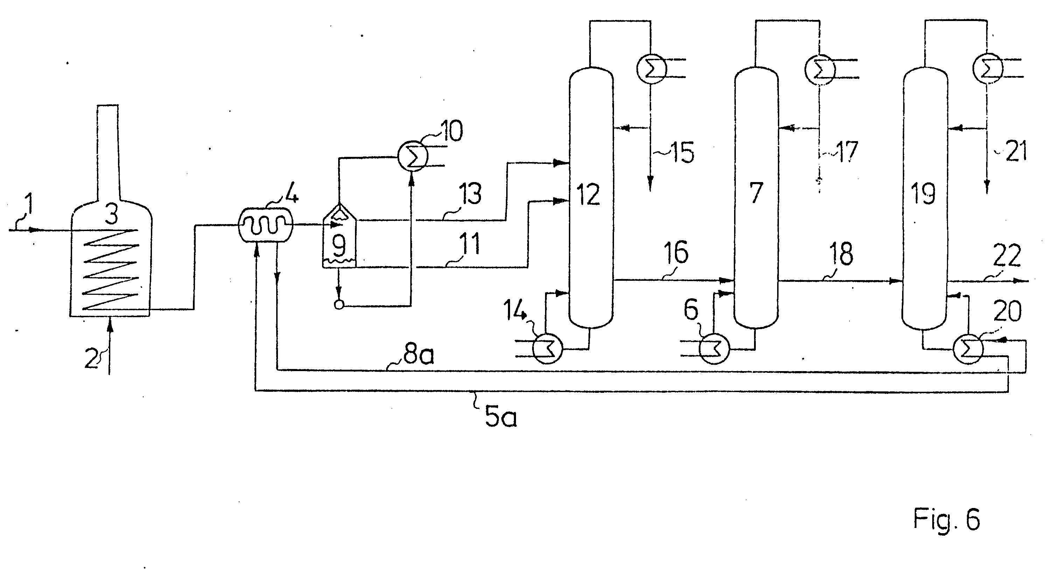

Man arbeitet gemäß dem Fließschema der Figur 6 und verfährt somit im wesentlichen wie im Beispiel 5. Das als Kühlmedium dienende Wasser für die mantelseitige Beschickung des Wärmetauschers (4) wird jedoch über Leitung (5a) dem Umlaufverdampfer (20) der Hochsiederkolonne (19) entnommen. Dieses Wasser hat eine Temperatur von 166°C und steht unter einem Druck von 7,2 bar. Es siedet bei 165—168°C und wird dampfförmig über die Leitung (8a) im Kreislauf in den Umlaufverdampfer (20) zurückgeführt. Der Spaltgasstrom wird im Wärmetauscher (4) von 510° auf 210°C abgekühlt, wobei eine Wärmemenge von 14 GJ/h entsprechend einer Heizdampfmenge von 6,7 t/h ausgetauscht wird.

Die Hochsieder-Kolonne (19) wird somit nicht mehr durch Heizdampf anlagenfremder Herkunft, sondern über die Leitungen (5a) und (8a) mit Hilfe der Pyrolyseenergie der Spaltgase betrieben. Gegenüber Figur 5 wird jedoch die Vinylchloridkolonne (7) jetzt durch den Umlaufverdampfer (6) mit von außerhalb der Anlage herangeführtem Heizdampf beheizt.

### Beispiel 7

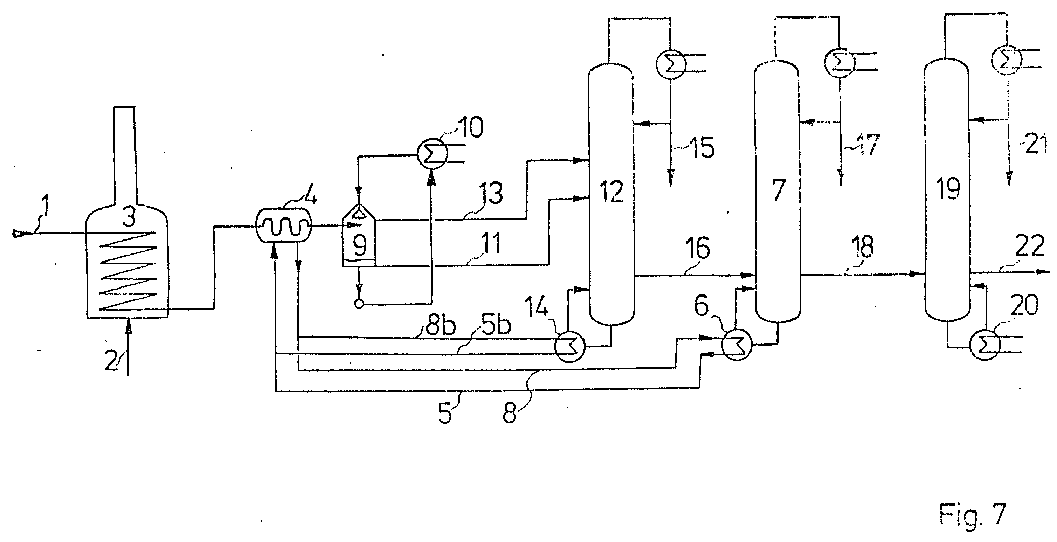

Man arbeitet gemäß dem Fließschema der Figur 7 und verfährt somit im wesentlichen wie im Beispiel 5. Jedoch wird zusätzlich der Umlaufverdampfer (14) der Chlorwasserstoffkolonne (12) über die Leitungen (5b) und (8b) an die Mantelleitungen 5 bzw. 8 des Wärmetauschers (4) angeschlossen. Entsprechend der erforderlichen erhöhten Heizleistung ist der Wärmetauscher (4) vergrößert. Im Wärmetauscher (4) werden die Spaltgase von 510° auf 200°C abgekühlt. Der gesamte Wärmeaustausch beträgt 13,7 GJ/h entsprechend einer Heizdampfmenge von etwa 6,5 t/h. Der Heizdampf hat eine Temperatur von 180°C bei 10 bar Druck; 2 t/h werden auf den Umlaufverdampfer (14) und 4,5 t/h auf den Umlaufverdampfer (6) verteilt. Die HCI-Kolonne (12) wird demnach nicht mehr durch Heizdampf anlagenfremder Herkunft, sondern über die Leitungen (5b) und (8b) mit Hilfe der Pyrolyseenergie der Spaltgase betrieben.

### Beispiel 8

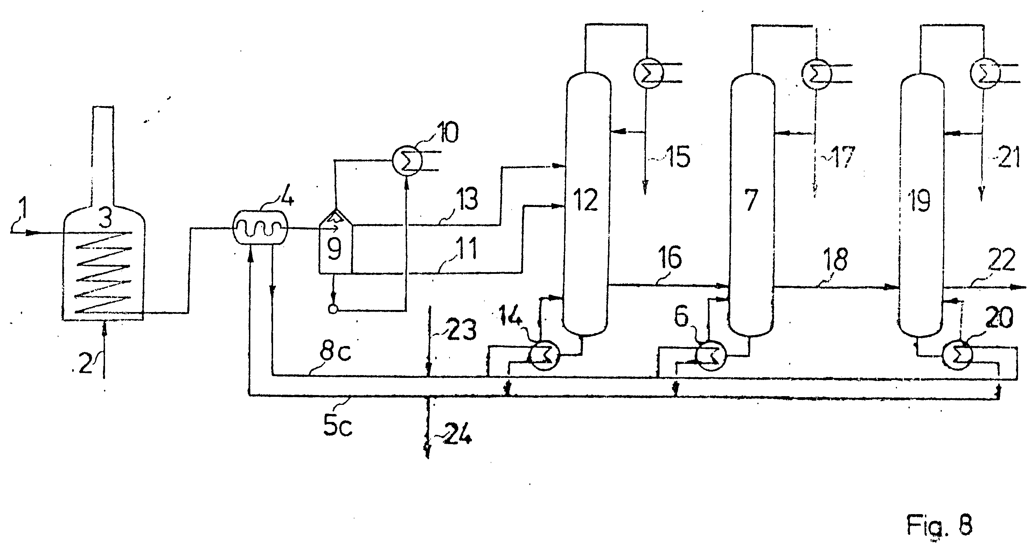

Man arbeitet gemäß dem Fließschema der Figur 8 und verfährt somit im wesentlichen wie im Beispiel 7. Jedoch wird an den Wärmetauscher (4) als 3. Verbraucher zusätzlich noch der Umlaufverdampfer (20) der Hochsiederkolonne (19) angeschlossen. Über die Verteilerleitung (5c) wird dem Mantelraum des Wärmetauschers (4) Wasser zugeführt, während über die Verteilerleitung (8c) Heizdampf von 10 bar und 180°C in die Umlaufverdampfer (14), (6) und (20) strömt. Über Leitung (23) kann zusätzlich benötigter Heizdampf aus einem fremden Netz eingespeist werden, während über Leitung (24) eine entsprechende Menge Wasser aus dem Kreislauf ausgeschleust werden kann.

### Beispiel 9

Man arbeitet gemäß dem Fließschema der Figur 5 und somit im wesentlichen wie im Beispiel 5. Jedoch wird der Wärmetauscher (4) mantelseitig mit über Leitung (5) vom Umlaufverdampfer (6) der Vinylchloridkolonne (7) als wärmeabführendem Kühlmedium herangeführtem Marlotherm[R]S (220 m³/h; 190°C) beschickt.

Hierdurch werden die Spaltgase auf 290°C (18 bar) abgekühlt, während das Kühlmedium auf 220°C aufgeheizt über Leitung (8) zum Umlaufverdampfer (6) zurückgeführt wird. Auf diese Weise wird im Wärmetauscher (4) eine Wärmemenge von 10,5 GJ/h ausgetauscht, was einer Heizdampfmenge von etwa 5 t/h entspricht, die bisher zum Betreiben der Kolonne (7) erforderlich war.

### Beispiel 10

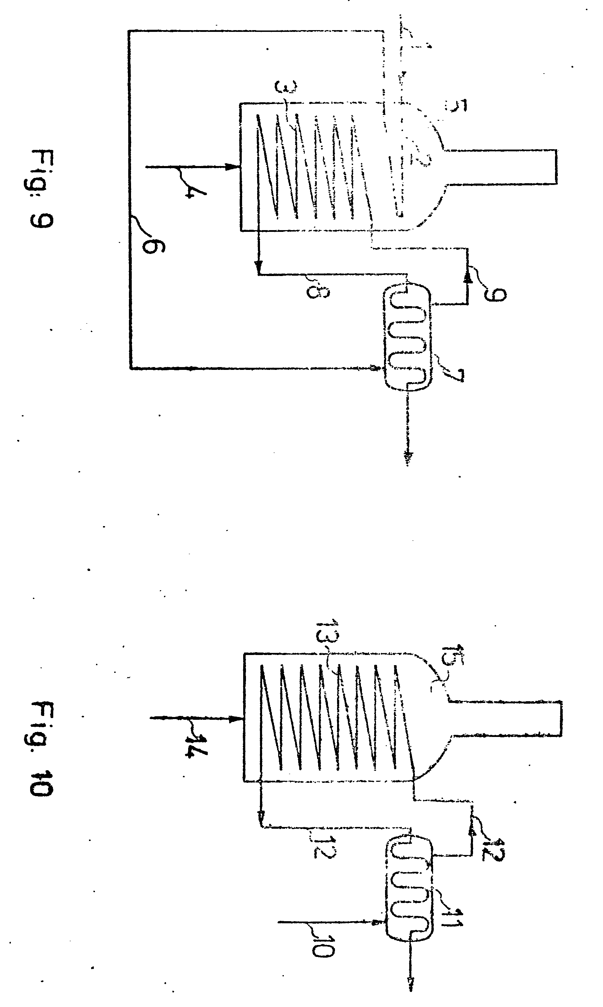

Man arbeitet gemäß dem Fließschema der Fig. 9.

Über Leitung (1) werden dem Vorwärmer (2), der sich oberhalb der Spaltzone (3) in dem gemeinsamen, mit Brenngas aus Leitung (4) beheizten Ofengehäuse (5) befindet, 38 000 kg/h flüssiges 1,2-Dichloräthan zugeführt und unter einem Druck von 32 bar auf 230°C aufgeheizt. Mit dieser Temperatur verläßt das noch flüssige 1,2-Dichloräthan den Ofen (5) und wird über Leitung (6) mantelseitig in den Wärmetauscher (7) geleitet. Produktseitig durchströmen den Wärmetauscher (7) 38 000 kg/h aus Leitung (8) herangeführtes Spaltgas mit einer Geschwindigkeit von 30 m/s, einer Temperatur von 510°C und einem Druck von 21 bar und werden auf 310°C abgekühlt, während auf der Mantelseite das als Kühlmittel wirkende 1,2-Dichloräthan verdampft und über Leitung (9) mit einer Temperatur von 260°C unter einem Druck von 30 bar in die Spaltzone (3) eintritt. Im Wärmetauscher (7) wird eine Wärmemenge von 8,4 GJ ausgetauscht. Der bisher erforderliche fremdbeheizte Verdampfer kann durch diesen Wärmetauscher ersetzt werden. Die teilweise abgekühlten Spaltgase werden in an sich bekannter Weise weiter abgekühlt und destillativ in ihre Bestandteile aufgetrennt.

### Beispiel 11

Man arbeitet gemäß dem Fließschema der Fig. 10.

Über Leitung (10) werden dem Wärmetauscher (11) mantelseitig 38 000 kg/h flüssiges 1,2-Dichloräthan unter einem Druck von 35 bar und mit einer Temperatur von 40° C als Kühlmittel zugeführt. Produktseitig durchströmen den Wärmetauscher (11) 38 000 kg/h aus Leitung (12) herangeführtes Spaltgas mit einer Geschwindigkeit von 30 m/s, einer Temperatur von 510° C und einem Druck von 21 bar und werden auf 170° C abgekühlt, während auf der Mantelseite das 1,2-Dichloräthan auf 250° C erwärmt wird, in einem (nicht eingezeichneten) Verdampfer verdampft und über Leitung (12) gasförmig in die Spaltzone (13) eintritt, die sich in dem mit Brenngas aus Leitung (14) beheizten Ofengehäuse (15) befindet. Im Wärmetauscher (11) wird eine Wärmemenge von 15,5 GJ/h ausgetauscht. Der in Beispiel 10 vorgesehene Vorwärmer (2) kann durch diesen Wärmetauscher ersetzt werden. Die teilweise abgekühlten Spaltgase werden in an sich bekannter Weise weiter abgekühlt und destillativ in ihre Bestandteile aufgetrennt.

### Patentansprüche

1. Verfahren zur Rückgewinnung von Pyrolyseenergie bei der Herstellung von Vinylchlorid, wobei man flüssiges 1,2-Dichloräthan unter Druck vorwärmt, verdampft und gasförmig einer Spaltzone zuführt, nach erfolgter unvollständiger thermischer Spaltung die unter Druck stehenden heißen Spaltgase kühlt und nacheinander in mehreren Destillationszonen zur Entfernung von Chlorwasserstoff, Vinylchlorid, ggf. Leichtsiedern und von Hochsiedern unter Rückgewinnung des nicht-umgesetzten 1,2-Dichloräthans in ihre Bestandteile auftrennt, dadurch gekennzeichnet, daß man die heißen Spaltgase in einem Wärmetauscher kühlt, der mantelseitig

a) vom Sumpf einer Destillationszone als Kühlmittel im Kreislauf durchströmt wird und dabei im Wärmetausch die betreffende Destillationszone heizt, oder

b) von einem im Kreislauf von einem Umlaufverdampfer mindestens einer Destillationszone herangeführten Wärmeübertragungsmittel durchströmt wird und dabei im Wärmeaustausch den Umlaufverdampfer mindestens einer Destillationszone beheizt, oder

c) von flüssigem 1,2-Dichloräthan als Kühlmittel durchströmt wird, das nach Wärmeaufnahme gasförmig der Spaltzone zugeführt wird.

2. Verfahren nach Anspruch 1, dadurch gekennzeichnet, daß man die heißen Spaltgase in mehreren hintereinandergeschalteten Wärmetauschern kühlt, die mantelseitig vom Sumpf je einer Destillationszone als Kühlmittel im Kreislauf durchströmt werden und dabei im Wärmeaustausch die betreffende Destillationszone heizen.

3. Verfahren nach Anspruch 1 oder 2, dadurch gekennzeichnet, daß das Sumpfprodukt einer Destillationszone den Wärmetauscher mantelseitig unter einem Druck von 1 – 15 bar mit etwa Siedetemperatur durchströmt, infolge Wärmeaufnahme seitens der Spaltgase verdampft und dampfförmig in die Destillationszone zurückströmt.

4. Verfahren nach Anspruch 1, dadurch gekennzeichnet, daß man als Wärmeübertragungsmittel Wasser einsetzt.

5. Verfahren nach Anspruch 1, dadurch gekennzeichnet, daß das vom Umlaufverdampfer einer Destillationszone herangeführte Wärmeübertragungsmittel Wasser ist, das den Wärmetauscher mantelseitig unter einem Druck von 5 bis 20 bar mit etwa Siedetemperatur durchströmt, infolge Wärmeaufnahme seitens der Spaltgase verdampft und dampfförmig zum Umlaufverdampfer der Destillationszone zurückströmt.

6. Verfahren nach Anspruch 1, dadurch gekennzeichnet, daß man ein Wärmeübertragungsmittel mit im Vergleich zu Wasser niedrigerem Dampfdruck und höherer kritischer Temperatur ( > 374,1° C) einsetzt.

7. Verfahren nach Anspruch 1, dadurch gekennzeichnet, daß man das zu spaltende flüssige 1,2-Dichloräthan unter einem Druck von 27 bis 36 bar auf 225 bis 255° C vorwärmt, noch flüssig als Kühlmittel in die Mantelzone des Wärmetauschers schickt und gasförmig unter einem Druck von 26 bis 35 bar mit einer Temperatur von 240 bis 260° C abzieht und der Spaltzone zuführt.

8. Verfahren nach Anspruch 1, dadurch gekennzeichnet, daß man das zu spaltende flüssige 1,2-Dichloräthan unter einem Druck von 30 bis 40 bar und mit einer Temperatur von 30 bis 50° C als Kühlmittel in die Mantelzone des Wärmetauschers schickt, dort vorwärmt, in einer anschließenden Verdampferzone verdampft und gasförmig unter einem Druck von 27 bis 36 bar mit einer Temperatur von 225 bis 255° C der Spaltzone zuführt.

9. Verfahren nach einem der Ansprüche 1 bis 8, dadurch gekennzeichnet, daß die heißen Spaltgase den oder die Wärmetauscher produktseitig mit einer Geschwindigkeit von mindestens 20 m/s durchströmen.

## Claims

1. Process permitting pyrolytic energy to be recovered in the production of vinyl chloride, wherein liquid 1,2-dichloroethane is preheated under pressure, evaporated and introduced in gaseous form into a cracking zone, subjected to an incomplete thermal cracking reaction therein, the resulting hot cracked gases maintained under pressure are cooled and seccessively separated into their constituents in a plurality of distilling zones for removal of hydrogen chloride, vinyl chloride, optionally low boilers and high boilers therefrom, and for recovery of unreacted 1,2-dichloroethane, characterized in that the hot cracked gases are cooled in a heat exchanger of which the jacket side

a) has material taken from the base portion of a distilling zone as a coolant passed therethrough, the respective distilling zone(s) being heated in heat exchange therewith; or

b) has a heat transfer agent passed therethrough, the heat transfer agent being recycled from the circulation evaporator associated with at least one of the distilling zones, the circulation evaporator of at least one distilling zone being heated by heat exchange therewith, or

c) has liquid 1,2-dichloroethane as a coolant passed therethrough which, after absorption of heat, is introduced in gaseous form into the cracking zone.

2. Process as claimed in claim 1, wherein the hot cracked gases are cooled inside a plurality of series-connected heat exchangers of which the jacket sides have material taken from the base portion of the respective distilling zones as a coolant circulated therethrough, the cracked hot gases heating the respective distilling zone by heat exchange.

3. Process as claimed in claim 1 or 2, wherein the base product taken from a distilling zone is passed through the jacket side of the heat exchanger under a pressure of 1 to 15 bars at about boiling temperature, evaporated by absorption of heat originating from the cracked gases and recycled in vapor form to the distilling zone.

4. Process as claimed in claim 1, wherein water is used as the heat transfer agent.

5. Process as claimed in claim 1, wherein the heat transfer agent coming from the circulation evaporator of a distilling zone is water, the water being passed through the jacked side of the heat exchanger under a pressure of 5 to 20 bars at about boiling temperature, evaporated by absorption of heat originating from the cracked gases and recycled in form of steam to the circulation evaporator of the distilling zone.

6. Process as claimed in claim 1, wherein the heat transfer agent used has a vapor pressure lower and a critical temperature higher than that of water ( > 374.1° C).

7. Process as claimed in claim 1, wherein the liquid 1,2-dichloroethane which is to be cracked is preheated under a pressure of 27 to 36 bars to 225 to 255° C, introduced, while liquid, into the jacket zone of the heat exchanger, removed in gas form therefrom under a pressure of 26 to 35 bars at a temperature of 240 to 260° C, and introduced into the cracking zone.

8. Process as claimed in claim 1, wherein the liquid 1,2-dichloroethane which is to be cracked is introduced under a pressure of 30 to 40 bars and at a temperature of 30 to 50° C into the jacket zone of the heat exchanger for use as a coolant therein, preheated therein, evaporated in an evaporation zone downstream of the heat exchanger, and introduced in gas form under a pressure of 27 to 36 bars and at a temperature of 225 to 255° C into the cracking zone.

9. Process as claimed in any of claims 1 to 8, wherein the hot cracked gases are passed through the product side(s) of the heat exchanger(s) at a velocity of flow of at least 20 m per second.

## Revendications

1. Procédé pour la récupération d'énergie de pyrolyse dans la préparation de chlorure de vinyle, dans lequel on préchauffe sous pression le 1,2-dichloréthane liquide, on l'évapore et on l'envoie à l'état gazeux dans une zone de dissociation, on refroidit les gaz de dissociation chauds sous pression après la dissociation thermique incomplète et on les sépare successivement en leurs constituants dans plusieurs zones de distillation pour l'élimination du chlorure d'hydrogène, du chlorure de vinyle, éventuellement des constituants de bas point d'ébullition et des constituants de haut point d'ébullition, avec récupération du 1,2-dichloroéthane non transformé, caractérisé en ce que l'on refroidit les gaz de dissociation chauds dans un échangeur de chaleur, dans lequel

a) on fait circuler comme agent réfrigérant dans l'enveloppe de l'échangeur le produit de pied de colonne d'une zone de distillation et on chauffe ainsi par échange de chaleur la zone de distillation correspondante, ou bien

b) on fait circuler dans l'enveloppe de l'échangeur un agent de transfert de chaleur recyclé d'un évaporateur à circulation d'au moins une zone de distillation et on chauffe ainsi par échange de chaleur l'évaporateur à circulation d'au moins une zone de distillation, ou bien

c) on fait circuler comme agent réfrigérant dans l'enveloppe de l'échangeur le 1,2-dichloroéthane liquide qui est envoyé à l'état gazeux après absorption de chaleur à la zone de dissociation.

2. Procédé selon la revendication 1, caractérisé en ce que l'on refroidit les gaz chauds de dissociation dans plusieurs échangeurs de chaleur disposés à la suite les uns des autres, dans lesquels on fait circuler du côté de l'enveloppe chaque fois le produit de pied de colonne d'une zone de distillation comme milieu réfrigérant et qui chauffent ainsi par échange de chaleur la zone de distillation correspondante.

3. Procédé selon la revendication 1 ou 2, caractérisé en ce que le produit de pied de colonne d'une zone de distillation passe dans l'échangeur de chaleur du côté de l'enveloppe sous une pression de 1 — 15 bars à peu près à la température d'ébullition, il s'évapore par suite le l'absorption de chaleur du côté des gaz de dissociation et retourne à l'état vapeur dans la zone de distillation.

4. Procédé selon la revendication 1, caractérisé en ce que l'on utilise l'eau comme agent de transfert de chaleur.

5. Procédé selon la revendication 1, caractérisé en ce que l'agent de transfert de chaleur provenant de l'évaporateur à circulation d'une zone de distillation est de l'eau qui traverse l'échangeur de chaleur du côté de l'enveloppe sous une pression de 5 à 20 bars à peu près à la température d'ébullition, s'évapore par suite de l'absorption de chaleur du côté des gaz de dissociation et retourne à l'état de vapeur à l'évaporateur à circulation de la zone de distillation.

6. Procédé selon la revendication 1, caractérisé en ce que l'on utilise un agent de transfert de chaleur ayant par rapport à l'eau une pression de vapeur plus faible et une température critique plus élevée ( $>374,1°C$ ).

7. Procédé selon la revendication 1, caractérisé en ce que l'on préchauffe le 1,2-dichloroéthane liquide à dissocier à 225 — 255°C sous une pression de 27 à 36 bars, on le charge encore liquide comme réfrigérant dans la zone d'enveloppe de l'échangeur de chaleur et on le prélève à l'état gazeux sous une pression de 26 à 35 bars à une température de 240 à 260°C et on l'envoie à la zone de dissociation.

8. Procédé selon la revendication 1, caractérisé en ce que l'on charge le 1,2-dichloroéthane liquide à dissocier sous une pression de 30 à 40 bars et à une température de 30 à 50°C comme réfrigérant dans la zone d'enveloppe de l'échangeur de chaleur, on l'y préchauffe, on l'évapore dans une zone d'évaporation montée à la suite et on l'envoie à l'état gazeux à une température de 225 à 255°C sous une pression de 27 à 36 bars dans la zone de dissociation.

9. Procédé selon l'une des revendications 1 à 8, caractérisé en ce que les gaz de dissociation chauds traversent l'échangeur de chaleur du côté du produit à une vitesse d'au moins 20 m/s.

Fig.1

Fig.2

Fig. 3

Fig. 4

Fig. 5

Fig. 6

Fig. 7

0 014 920

Fig. 8

Fig. 9

Fig. 10